# EUROPEAN PATENT SPECIFICATION

(11) **EP 0 336 964 B1**
(45) Date of publication and mention of the grant of the patent: **04.08.1993**
(21) Application number: 87907978.8
(22) Date of filing: 02.12.1987
(51) Int. Cl.: A61L 33/00, C08B 37/10

(54) **ANTITHROMBOTIC MEDICAL MATERIALS AND PROCESS FOR THEIR PRODUCTION**
ANTITHROMBOTISCHE ARZNEIMITTEL UND DEREN HERSTELLUNG
SUBSTANCES MEDICINALES ANTITHROMBOTIQUES ET PROCEDE DE PRODUCTION

(30) Priority: 03.12.1986 JP 286661/86; 19.12.1986 JP 301422/86
(43) Date of publication of application: 18.10.1989
(73) Proprietor: TERUMO KABUSHIKI KAISHA, Tokyo 151 (JP)
(72) Inventor: KASHIWAGI, Nobuyoshi, Fuji-shi Shizuoka 417 (JP); SASAKI, Masatomi Terumo Kabushiki Kaisha, Shizuoka 417 (JP); SAKAKIBARA, Hiroki Terumo Kabushiki Kaisha, Fuji-shi Shizuoka 417 (JP); HAGIWARA, Kazuhiko Terumo Kabushiki Kaisha, Fuji-shi Shizuoka 417 (JP)
(74) Representative: Gillard, Marie-Louise
(86) International application number: JP8700934
(87) International publication number: WO8804183

(56) References cited:
- EP-A- 0 212 933
- WO-A-87/06007
- DE-A- 3 109 141

## Description

### Technical Field

The present invention relates to antithrombotic medical materials and a process for preparing the same. More particularly, it is concerned with antithrombotic medical materials comprising heparin bonded via an epoxy compound with a high molecular compound and a process for preparing the same.

Anticoagulant activity of the heparin bonded with a high molecular compound remains intact and stable for a long period of time, and therefore the antithrombotic medical materials of the invention is especially useful as medical materials which may be used in contact with blood.

### Background Art

A method of providing medical materials with antithrombotic activity by the use of heparin has hitherto been known. For example, Japanese Patent Laid-Open-to-Public Sho 58-10053 discloses a process in which heparin is bonded with medical materials by forming a covalent bond. According to said process, however, the active site of heparin also may participate in the covalent bonding to anticipate the loss of the anticoagulant activity and subsequent occurrence of thrombosis, and so the process is undesirable. As disclosed in Japanese Patent Laid-Open-to-Public sho 49-8583 and sho 49-44590, there are also known processes in which heparin is bonded by an ionic bond. The products of these processes are not suitable for use over a long period of time, because the bond between the heparin and the medical material is so weak that heparin is readily decomposable. Antithrombogen materials are also known which comprise heparin bound to a compound containing carboxyl, sulfonate or hydroxyl groups via a spacer (see DE-A-3 109 141).

### Disclosure of the Invention

The present invention is directed to overcoming the problems in the prior art as pointed out above. It is therefore an object of the invention to provide antithrombotic medical materials in which anticoagulant activity of heparin is maintained and solid bonding is formed between a high molecular compound such as cellulose and heparin and a process for preparing the same.

Another object of the present invention is to provide heparin derivatives useful as an intermediate for the above-mentioned antithrombotic medical materials and a process for preparing the same.

In order to achieve the above objects, the present invention is composed of:
1. A process for preparing an antithrombotic medical material comprising heparin bonded via an epoxy compound with two or more epoxy groups with a high molecular compound containing a functional group capable of bonding with an epoxy group wherein an amino group in the heparin is bonded with the epoxy group in the epoxy compound and the functional group capable of bonding with the epoxy group in the high molecular compound is bonded with the epoxy group in the epoxy compound, which comprises:
   a) reacting the amino group in heparin with the epoxy group in the epoxy compound in an aqueous solution at a pH not lower than 7; and
   b) reacting the functional group capable of bonding with an epoxy group in the high molecular compound with an epoxy group in the heparin derivative obtained in step a), said heparin derivative being beforehand immersed in a liquid medium, unreacted epoxy group(s) remaining in the reaction product.
2. The process for preparing the antithrombotic medical material according to item 1, wherein the functional group capable of bonding with the epoxy group is at least any one of a hydroxyl group, an amino group, a carboxyl group and a mercapto group.
3. The process for preparing the antithrombotic medical material according to item 1 or 2, wherein the liquid medium is a buffer solution.
4. The process for preparing the antithrombotic medical material according to item 3, wherein the buffer solution is an aqueous phosphate or borate solution.
5. The process for preparing the antithrombotic medical material according to item 1, wherein the liquid medium is an aqueous solution of an inert hydrophilic organic solvent.
6. A process for preparing a heparin derivative produced by reacting heparin with an epoxy compound containing two or more epoxy groups comprising a bond between the amino group of the heparin and the epoxy group of the epoxy compound, unreacted epoxy groups remaining in the reaction product, which comprises reacting heparin with an epoxy compound with two or more epoxy groups for 4 days at RT in a liquid medium at a pH not lower than 7, then neutralizing the mixture with hydrochloric acid and removing the unreacted epoxy groups by extraction.
7. The process according to item 6, wherein the pH is between 8 and 10.
8. The process according to item 6, wherein the reaction is conducted in an aqueous solution at pH9.
9. The process according to item 6, wherein the epoxy compound is 1,4-butanediol diglycidyl ether.

Heparin used in the present invention is a mucopolysaccharide possessing an anticoagulant activity and is widely distributed in the muscles, lungs, spleen and liver. It is used for the therapy of thrombosis. As mentioned above, a large number of attempts to coat a variety of medical materials with heparin have been made in order to prevent formation of thrombus when used in the living body.

It is required for the epoxy compounds employed in the invention to possess two or more epoxy groups. As preferable examples there may be mentioned glycidyl compounds, and 1,4-butanediol diglycidyl ether and polyether diglycidyl ethers (e.g., polyethylene glycol diglycidyl ethers) are preferred. Particularly preferred is 1,4-butanediol diglycidyl ether.

The high molecular compounds employed in the invention are preferably those which contain at least any one of a hydroxyl group, an amino group, a carboxyl group and a mercapto group. As examples of such high molecular compound are mentioned cellulose, cross-linked polyvinyl alcohol, poly(ethylene-vinyl alcohol) copolymer and polyhydroxyethyl methacrylate as the hydroxyl group-containing one, chitosan, fibroin, polyvinylamine, polyacrylamide and collagen as the amino group-containing one, and polymethacrylic acid, polyacrylic acid and chitin as the carboxyl group-containing one. Cellulose is especially preferred.

The process for preparing the antithrombotic medical materials according to the present invention comprises carrying a step of reacting the amino group of heparin with the epoxy group of an epoxy compound and a step of reacting the functional group capable of reacting with epoxy group of a high molecular compound with the epoxy group of an epoxy compound. The former step is conducted by combining heparin and an epoxy compound in solution. The heparin and the epoxy compound are employed desirably at a molar ratio between ca. 1:4 and 1:10. The reaction solution should be maintained at a pH of not lower than 7, preferably between 7 and 12 and more preferably between 8 and 10. At a pH below 7, not only the sulfamino group of heparin will possibly be destroyed with a result that the heparin would lose the antithrombotic activity but also the epoxy compound will possibly undergo cleavage of the epoxy group, so that the reaction would no longer take place.

The reaction is carried out at a temperature above the solidifying temperature of the reaction solution but not higher than 150°C. A temperature above 150°C possibly collapses heparin molecules and is not applicable.

If both pH and temperature are high in the reaction, reactivity will be too high to cause bonding of all the epoxy groups present in the epoxy compound with heparin so that not only the bonding with the high molecular medical material will be impossible but also there may occur formation of the bonds with groups other than amino group in heparin and subsequent reduction in potency of the heparin and also decomposition of the heparin itself. Accordingly, the reaction temperature should be lower when the pH is higher, and the pH should be reduced when the reaction temperature is high.

The reaction time in general is from 30 min. to 10 days though it is variable depending upon reaction conditions such as pH and temperature. For example, the reaction is suitably carried out at room temperature for 4 days when the pH is 9.

The reaction is preferably carried out in the presence of an aqueous solution, that is, water, an aqueous buffer solution or an aqueous solution of an inert hydrophilic organic solvent. More preferably, an aqueous solution of sodium hydroxide or potassium hydroxide, a phosphate or borate buffer solution, a mixture of a tetrahydrofuran solvent and water (for example, tetrahydrofuran : water = 9 : 1), a mixture of a dioxane solvent and water (for example, dioxane : water = 3 : 1) and the like are employed. By adopting moderate reaction conditions as described above, only the amino group that does not directly participate in development of the anticoagulant activity of heparin can be reacted with epoxy group, and bonding of all the epoxy groups present in the epoxy compound can be prevented.

The heparin derivatives thus obtained preferably contain 1-10 unreacted epoxy groups per molecule.

Next, the reaction between a functional group capable of reacting with an epoxy group in the high molecular compound and an epoxy group in the epoxy compound or the reaction between the high molecular compound and the product obtained in the above step in which the epoxy compound and heparin are bonded (heparin derivative) is carried out by contacting the high molecular compound with the epoxy compound or the heparin derivative in a liquid medium, preferably an aqueous solution at a pH not lower than 7. It is desirable that the epoxy group and the functional group capable of bonding therewith is equal in number. The aqueous solution referred to has the same meaning as defined above.

When the functional group in the high molecular compound is an amino group which is highly reactive with an epoxy group, pH of the reaction solution is not lower than 7, preferably between 7 and 12 and more preferably between 8 and 10. When the functional group in the high molecular compound is a group other than an amino group, that is, a hydroxyl group, a carboxyl group or a mercapto group, it is desirable to alkalinize the high molecular compound in advance to activate it in order to distinguish the above-mentioned group in reactivity from the hydroxyl group and the carboxyl group in the heparin derivative. The alkalinized compound is then reacted with the heparin derivative. As a matter of fact, the above-mentioned high molecular compound is immersed in an aqueous alkali solution to activate the former, which is then immersed in a solution of the heparin derivative for the reaction. In said reaction, diffusion of the alkali from the above-mentioned activated high molecular compound to the reaction solution can be inhibited by using an aqueous solution of an inert hydrophilic organic solvent as described above. This can then result in maintenance of the activity as well as prevention of pH rise and reduction in heparin activity. As the inert hydrophilic organic solvent, dioxane and tetrahydrofuran are suitable. Ratio of the solvent to water is dioxane:water = 1:10 - 4:1, preferably 2:1 - 3:1 and tetrahydrofuran:water = 1:10 - 10:1, preferably 7:1 - 9:1. The aqueous alkali solution is prepared preferably by the use of sodium hydroxide or potassium hydroxide, concentration of the alkali being 0.01% - 35%, preferably 0.1% - 10%.

When a buffer solution is employed as the solvent, an aqueous phosphate or borate solution is preferable, for example, of potassium dihydrogen phosphate (KH₂PO₄)-sodium hydroxide (NaOH), boric acid (H₃BO₃)-potassium chloride (KCl) or potassium dihydrogen phosphate (KH₂PO₄)-disodium hydrogen phosphate (Na₂HPO₄). If pH is below 7 during the reaction, activity of the heparin could unfavorably disappear due to reactions such as heparin decomposition. If pH is higher than 12 during the reaction, the reactivity could be so high as to cause a reaction between the epoxy compounds (or the heparin derivatives) and the number of epoxy groups to be reacted with the high molecular compound or the heparin activity would unfavorably be reduced. The reaction temperature is in the range from such a temperature as not solidifying the reaction solution to 150°C. At a temperature higher than 150°C the heparin derivative could unfavorably be collapsed.

The reaction time is about from 5 min. to 10 days though it is variable depending upon nature of the high molecular compound and other reaction conditions.

Whereas the above two steps may be carried out in any order, it is preferable in view of simpler operation that a heparin derivative is first prepared and then the high molecular compound is immersed in an aqueous solution of the heparin derivative to cause reaction between an epoxy group of the heparin derivative and a group such as a hydroxy group of the high molecular compound. The latter step may be by an operation other than immersion and be any operation that can cover an aqueous solution of the heparin derivative over the surface of the high molecular compound. In the antithrombotic medical materials of the invention thus obtained, heparin is firmly bonded with a high molecular compound via an epoxy compound and also the bond is stable, so that they are advantageously used as a medical material which will be used in contact with blood such as artificial blood vessel, artificial organ indwelling catheter in the blood vessel, blood filter or plasma separator.

### Examples

The invention will be described in more details by means of illustrative exmaples.

### Example 1.

A solution of 1.00 g of sodium heparin (188 IU/mg) and 0.018 g of 1,4-butanediol diglycidyl ether (manufactured by Aldrich) in 10 ml of distilled water was adjusted with 0.1 N aqueous sodium hydroxide solution to pH 9 and reacted at room temperature for 4 days. After completion of the reaction, neutralization was made with 0.01 N hydrochloric acid, followed by extraction with ether to remove excess 1,4-butanediol diglycidyl ether. There was produced aqueous solution of a heparin derivative (called heparin macromer hereinbelow).

A piece of regenerated cellulose sheet (manufactured by ENKA) of a surface area of 6 cm² was immersed in 0.5% aqueous solution of sodium hydroxide for 30 min. After swabbing up the aqueous sodium hydroxide solution attached to the regenerated cellulose sheet, the sheet was immersed in 3 ml of 0.2% aqueous solution of the heparin macromer prepared as above. After immersed for a predetermined period of time, the resulting sheet was washed with water to give a heparin-fixed regenerated cellulose sheet. Hydrogen ion concentration of the reaction solution used was measured.

### Example 2.

A piece of regenerated cellulose sheet (manufactured by ENKA) (surface area: 6 cm²) was immersed in 0.5% aqueous solution of sodium hydroxide for 30 min. After swabbing up the aqueous sodium hydroxide solution attached to the regenerated cellulose sheet, the sheet was immersed in 3 ml of potassium dihygrogen phosphate-sodium hydroxide buffer solution (adjusted to pH 8) containing 0.2% of the heparin macromer prepared in the same way as in Example 1. After immersed for a predetermined period of time, the resulting sheet was washed with water to give a heparin-fixed regenerated cellulose sheet. Hydrogen ion concentration of the reaction solution used was measured.

### Example 3.

A piece of regenerated cellulose sheet (manufactured by ENKA) (surface area: 6 cm²) was immersed in 0.5% aqueous solution of sodium hydroxide for 30 min. After swabbing up the aqueous sodium hydroxide solution attached to the regenerated cellulose sheet, the sheet was immersed in 3 ml of boric acid-potassium chloride-sodium hydroxide buffer solution (adjusted to pH 8) containing 0.2% of the heparin macromer. After immersed for a predetermined period of time, the resulting sheet was washed with water to give a heparin-fixed regenerated cellulose sheet. Hydrogen ion concentration of the reaction solution used was measured.

### Example 4.

A piece of regenerated cellulose sheet (manufactured by ENKA) (surface area: 6 cm²) was immersed in 0.5% aqueous solution of sodium hydroxide for 30 min. After swabbing up the aqueous sodium hydroxide solution attached to the regenerated cellulose sheet, the sheet was immersed in 3 ml of boric acid-potassium chloride-sodium hydroxide buffer solution (adjusted to pH 9) containing 0.2% of the heparin macromer. After immersed for a predetermined period of time, the resulting sheet was washed with water to give a heparin-fixed regenereted cellulose sheet. Hydrogen ion concentration of the reaction solution used was measured.

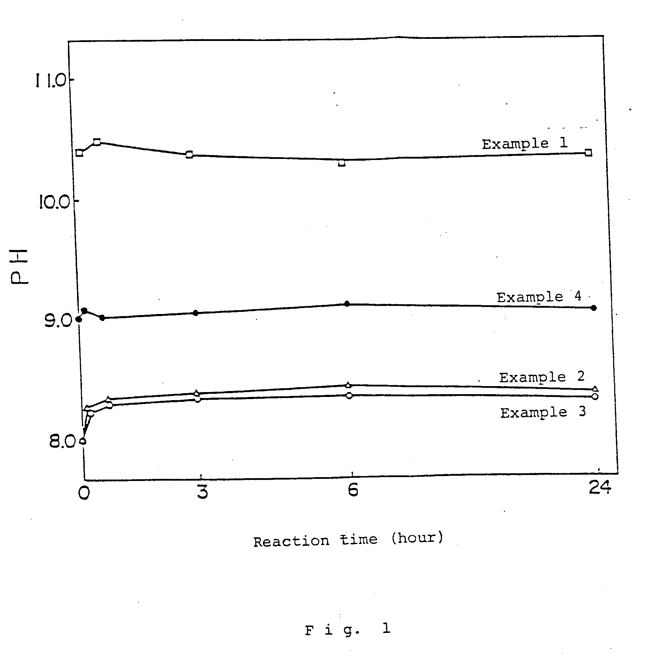

Changes with time in the hydrogen ion concentration of the reaction solution measured in Examples 1, 2, 3 and 4 are shown in Fig. 1. The results indidate indicate that hydrogen ion concentration of the reaction solution for which a buffer solution is used (Examples 2-4) changes to a less degree from that of the reaction solution at the start of reaction than the hydrogen ion concentration of the reaction solution for which an aqueous solution is used. In other words, the reaction can be effected under more stable conditions by the use of a buffer solution thereby enabling efficient operation without fear of reducing the heparin activity.

Then, heparin activity was determined by adding Antithrombin III to the reaction solution followed by reaction with a predetermined excess of Factor-Xa and quantitatively assaying activity of the remaining Factor-Xa with a color-developing synthetic substrate (s-2222). Results are shown in Table 1. Comparative value was determined by taking the initial amount of heparin prepared (theoretical value) as 100 and expressing the heparin activity in the reaction solution in percentage. The results indicate that no heparin activity was lost.

**Table 1**

| | Example 1 | Example 2 | Example 3 | Example 4 |
|---|---|---|---|---|
| Absorbancy (A 405) | 0.410 | 0.408 | 0.424 | 0.404 |
| Heparin potency (IU/ml) | 0.40 | 0.40 | 0.39 | 0.41 |
| Comparative value (%) | 100 | 100 | 98 | 103 |

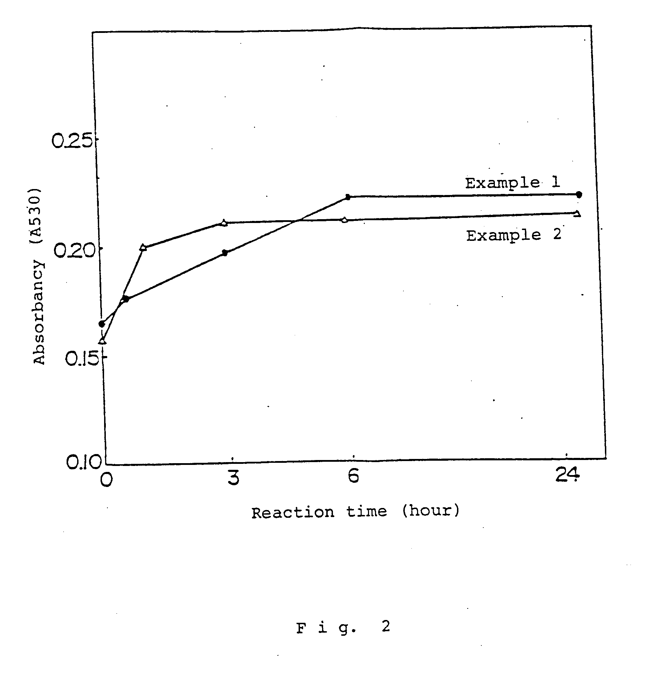

Next, amount of the heparin fixed on the heparin-bonding regenerated cellulose sheet (got by Examples 1 and 2) was determined by Toluidineblue-O method. Results are shown in Fig. 2. The results indicate that amount of the heparin fixed on the heparin-bonding regenerated cellulose remains almost constant after 6 hours.

### Example 5.

1) 1% chitosan solution was prepared by dissolving chitosan in formic acid, and a chitosan sheet was prepared on a Teflon plate by casting.
2) The chitosan sheet (surface area: 6 cm²) prepared under 1) above was immersed for 24 hours in 10 ml of the phosphate buffer solution (potassium dihydrogen phosphate-sodium hydroxide: adjusted to pH 8) containing 0.2% of the heparin macromer prepared in Example 1.
3) Then, the treated chitosan sheet was thoroughly washed with the phosphate buffer solution (adjusted to pH 8).

### Comparative Example 1.

1) A chitosan sheet was prepared in the same way as under 1) of Example 5.
2) The chitosan sheet (surface area: 6 cm²) prepared under 1) above was immersed in 10 ml of the phosphate buffer solution (adjusted to pH 8) containing 0.2% of heparin.
3) A chitosan sheet treated in the same way as under 3) of Example 5 was prepared.

Amounts of the heparin fixed on the chitosan sheets prepared according to Example 5 and Comparative Example 1 were determined by the staining method with Toluidine Blue-O as described below.
1) Each of the chitosan sheets (surface area: 6 cm² each) is immersed in 5 ml of 0.04% Toluidine Blue-O for 10 min.
2) It is thoroughly washed with the phosphate buffer solution (adjusted to pH 8).
3) The chitosan is immersed in 50 ml of a color-developing solution (ethanol : 0.1 N aqueous solution of sodium hydroxide = 4 : 1) for 20-30 min. followed by measurement of absorbance at 530 nm.

- Note): No washing with 0.1 N hydrochloric acid (different from the proior-art method)
Amount of the heparin on the treated chitosan sheet as determined by Toluidineblue-O method is shown in Table 2. The results show that heparin is bonded with the chitosan sheet in the present invention.

**Table 2**

| | | Example 5 | Comparative Example 1 |
|---|---|---|---|
| Absorbancy (530 nm) | (1) | .280 | .164 |
| | (2) | .298 | .174 |
| | (3) | .254 | .168 |

### Example 6.

A solution of 1.00 g of sodium heparin (188 IU/mg) and 0.018 g of 1,4-butanediol diglycidyl ether (manufactured by Aldrich) in 10 ml of distilled water was adjusted with o.1 N aqueous sodium hydroxide solution to pH 9 and reacted at room temperature for 4 days. After completion of the reaction, neutralization was made with 0.01 N hydrochloric acid, followed by extraction with ether to remove excess 1,4-butanediol diglycidyl ether. There was produced aqueous solution of a heparin derivative.

A regenerated cellulose sheet (manufactured by ENKA) (surface area: 250 cm²) was immersed in 0.5% aqueous solution of sodium hydroxide for 30 min. After swabbing up the aqueous sodium hydroxide solution attached to the regenerated cellulose sheet, the sheet was immersed for 3 days in each (70 ml) of 0.2% aqueous solution, dioxane-water (3 : 1) solution and tetrahydrofuran-water (9 : 1) solution of the heparin macromer prepared as above. The regenerated cellulose was then removed, and hydrogen ion concentration of the reaction solution was determined. (In the case where an organic solvent-water system was employed, the organic solvent was dried in an evaporator followed by dilution with an equal amount of distilled water.)

Changes in the hydrogen ion concentration of the reaction solution determined in Example 6 are shown in Table 3. It is seen that use of an organic solvent in the dioxane-water system and the tetrahydrofuran-water system results in better inhibition of the dissolution of alkali out of the above-mentioned high molecular compound than the water system.

**Table 3**

| | Water system | Dioxane/water system | Tetrahydrofuran/water system |
|---|---|---|---|
| pH | 11.25 | 9.08 | 9.16 |

### Example 7.

A piece of regenerated cellulose sheet (manufactured by ENKA) (surface area: 6 cm²) was immersed in 0.5% aqueous solution of sodium hydroxide for 30 min. Then, after swabbing up the aqueous sodium hydroxide solution attached to the regenerated cellulose sheet, the resulting sheet was immersed in each of 0.2% aqueous solution and dioxane-water (3 : 1) solution of the heparin macromer prepared according to Example 6. After immersed at room temperature for 24 hours, the sheet was washed with water. There was produced a heparin-fixed regenerated cellulose sheet.

Amount of the heparin fixed on the heparin-fixed regenerated cellulose sheet thus obtained was determined by Toluidineblue-O method.
1) A piece of the cellulose (surface area of 6 cm²) is placed in a test tube in which is then placed 0.04% Toluidine Blue solution, and the test tube is allowed to stand for 5 min.
2) Decantation washing is made with two portions of 10 ml of distilled water.
3) To the test tube is added 5 ml of 0.1 N hydrochloric acid, followed by allowing to stand for 10 min. and decantation. The same treatment with 5 ml of 0.1 N hydrochloric acid is repeated.
4) Then, 10 ml of distilled water is added followed by allowing to stand for 10 min. and decantation. The same treatment with 10 ml of distilled water is repeated.
5) Water drops on the surface of the cellulose piece is removed. Six pieces of the cellulose prepared as above are placed in a test tube.
6) In the test tube is then placed a buffer solution (ethanol and 0.1 N aqueous solution of sodium hydroxide in a volume ratio of 4 : 1), and after 20-30 min. absorbance is measured at 530 nm.

**Table 4**

| | Comparative control (cellulose) | Treated cellulose (water ststem) | Treated cellulose (dioxane/water system)* |
|---|---|---|---|
| Amount of heparin(absorbancy 530 nm) | 0.132 | 0.222 | 5.933 |

| | | | |
|---|---|---|---|
| (* 1 : 10 diluted and measured) | | | |

Results of the absorbancy measurement are shown in Table 4. It is seen from Table 4 that a marked increase in the bonded amount of heparin is observed by changing the reaction solution to be treated from water system to organic solvent/water system.

### Example 8.

To a solution of 0.081 g (4 x 10⁻⁴ mol) of 1,4-butanediol diglycidyl ether in 10 ml of water was added 1.0 g of sodium heparin. After producing a homogeneous solution, pH of the reaction solution was adjusted with 0.1 N sodium hydroxide solution to 9. The solution was stirred at room temperature for 4 days, and the pH was adjusted with 0.1 N hydrochloric acid to 7.0. The resulting solution was then extracted with three portions of 10 ml of diethyl ether in order to remove unreacted 1,4-butanediol diglycidyl ether. Residual aqueous solution of the heparin derivative was slightly evaporated and then diluted with water to a predetermined amount.

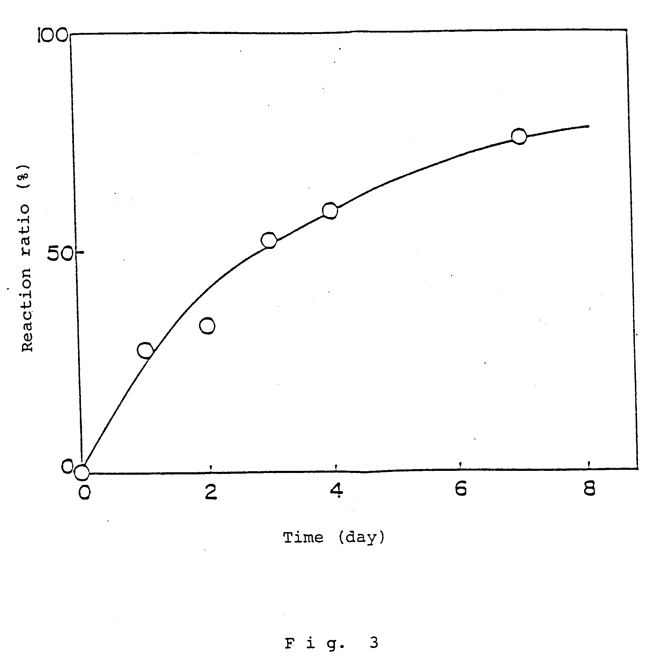

Decreased amount of the 1,4-butanediol diglycidyl ether during preparation of the heparin derivative as described above was examined by high performance liquid chromatography. As shown in Fig. 3, the reaction ratio reached 60% on the 4th day of the reaction. On the other hand, it has been reported that 3-4 amino groups are present in the starting heparin (U. Lindahl et al. J. Biol. Chem., 248, 7234 (1973)) and this was confirmed for the present heparin by the ninhydrin method. Molecular weight of the heparin was determined to be 13000 as measured by the light scattering method.

These data indicate that ca. 3 epoxy groups are present per molecule of the heparin derivative prepared according to the invention.

Amount of the amino group contained in the heparin derivative prepared as above was also determined according to the ninhydrin method as described below.
1) In 75 ml of methylcellosolve are dissolved 2.0 g of ninhydrin and 0.3 g of hydrindantin. To the solution is then added 25 ml of 4 N sodium acetate buffer, and the mixture is used as ninhydrin reagent.
2) Heparin and 1% aqueous solution of the heparin derivative prepared as above are placed respectively in a volume of 0.75 ml in a test tube.
3) To the test tube is then added 0.5 ml of the ninhydrin reagent prepared under 1). The mixture is stirred and reacted at 100°C for 15 min.
4) After cooling, 5 ml of 50% aqueous ethanol solution is added immediately followed by measurement of absorbance at 570 nm.

Results are shown in Table 5.

**Table 5**

| | Heparin | Heparin derivative |
|---|---|---|
| Amount of amino group (calculated in terms of leucin) | 0.102 µmol/ml | 0.02 µmol/ml |
| Relative proportion of amino group | 100% | 19.6% |

As shown in Table 5, conversion of heparin to the heparin derivative of the invention leads to disappearance of most of the amino groups thereby demonstrating the specific reaction between the epoxy group and the amino group.

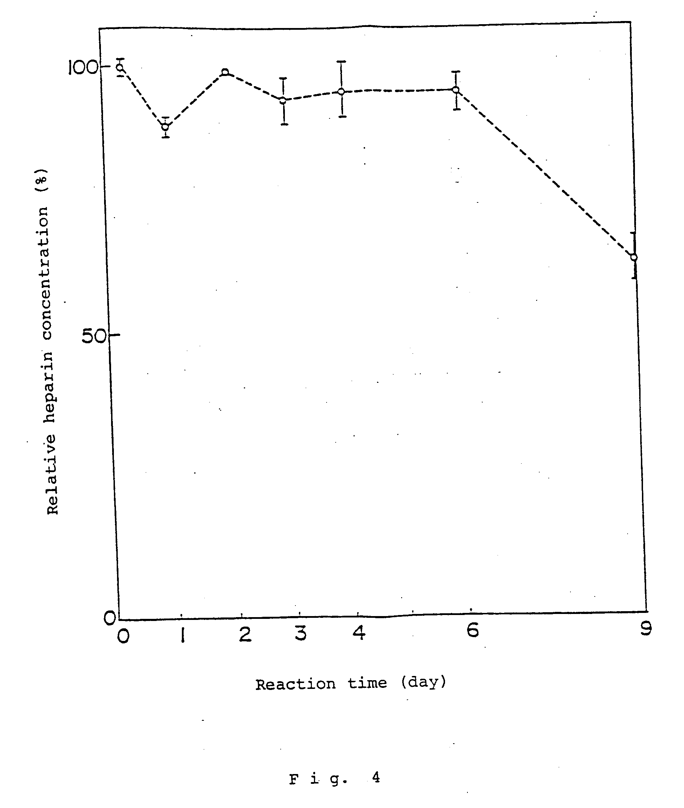

Heparin activity during the reaction for preparing the heparin derivative was followed up to the 9th day. As shown in Fig. 4, there was no change of the activity observed until the 6th day.

Moreover, relationship between the amount of heparin bonded to cellulose and the reaction time for preparing the heparin macromer was examined. As shown in Frg. 5, the maximum amount of heparin bonded with cellulose was observed on the 4th day of the reaction.

### Example 9. Bonding treatment between the heparin derivative and cellulose

A piece of cellulose sheet (surface area: 3 cm²) was immersed in 0.5% aqueous solution of sodium hydroxide for 30 min. The alkali-treated piece of cellulose was taken out, from which the aqueous sodium hydroxide solution was gently removed. Three pieces of the cellulose prepared as above were immersed in 10 ml of 0.2% aqueous solution of the heparin derivative (375.8 IU/ml), thoroughly washed after a predetermined period of time and measured for the amount of heparin bonded.

Measurement of heparin is made by using the improved Ortho-Toluidineblue-O method as follows:
1) The cellulose piece is placed in a test tube followed by addition of 0.04% solution of Toluidine Blue, and the mixture is allowed to stand for 5 min.
2) Decantation washing is made with two portions of 10 ml of distilled water.
3) To the test tube is added 5 ml of 0.1 N hydrochloric acid, and the mixture is allowed to stand for 10 min. After decantation, the treatment with 5 ml of 0.1 N hydrochloric acid is repeated in the same way as above.
4) To the test tube is added 10 ml of distilled water, and the mixture is allowed to stand for 10 min. After decantation, the treatment with 10 ml of distilled water is repeated in the same way as above.
5) Water drops on the cellulose piece are removed, and six pieces of the cellulose prepared as above are placed in a test tube.
6) To the test tube is then added a buffer solution (4 : 1 in volume mixture of ethanol and 0.1 N aqueous solution of sodium hydroxide). After 20-30 min., absorbance at 530 nm is measured.

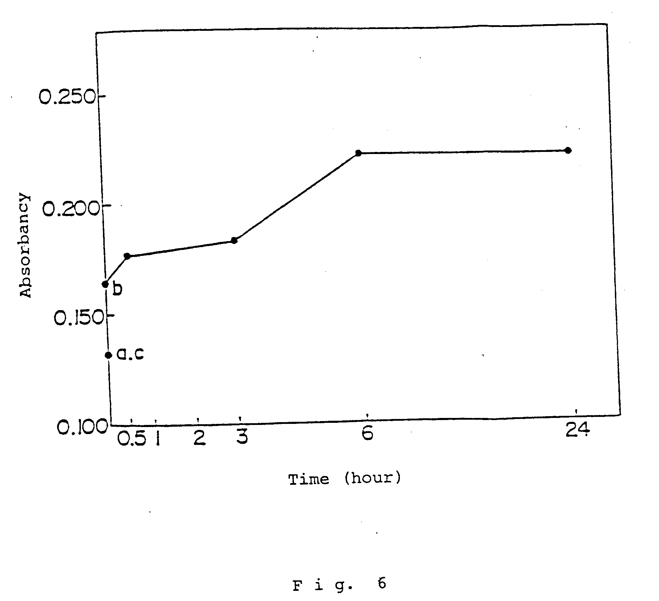

Results of the absorbancy measurement are shown in Fig. 6. As shown in Fig. 6, cellulose and the heparin derivative produced a constant amount of heparin bonded after 6 hours from initiation of the reaction.

### Brief Description of the Drawing

Fig. 1 is a graphic presentation of the hydrogen ion concentration in the reaction solution used in the invention.

Fig. 2 is a graphic presentation of the amount of Toluidineblue-O that stained the heparin-fixed regenerated cellulose sheet in the antithrombotic medical material produced in the present invention.

Fig. 3 is a graphic presentation of the reaction ratio between heparin and 1,4-butanediol diglycidyl ether.

Fig. 4 is a graphic presentation of the change in heparin activity during preparation of the heparin derivative.

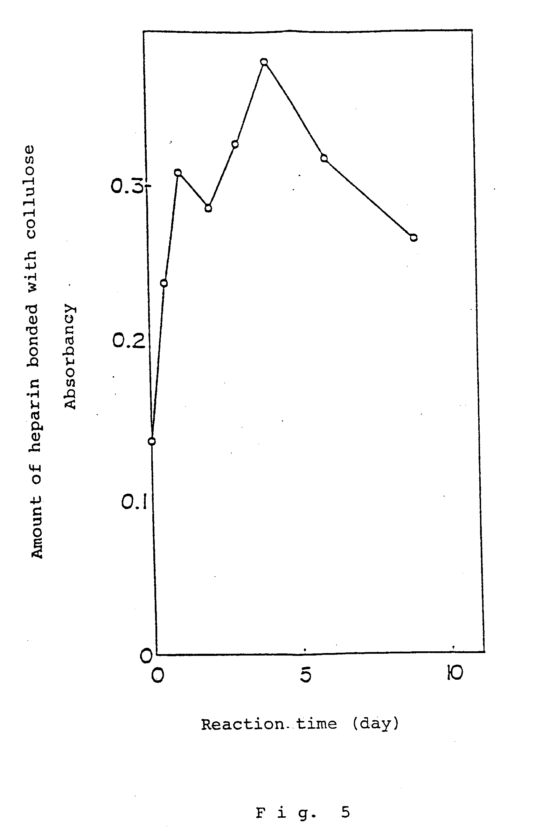

Fig 5 is a graphic presentation of relationship between the amount of heparin bonded to cellulose and the reaction time for preparing the heparin macromer.

Fig. 6 is a graphic presentation of the reaction time and the amount bonded between cellulose and the heparin derivative.

### Possible Industrial Utility

The antithrombotic medical material according to the present invention are favorably used as materials for a variety of medical instruments to be contacted with blood and artificial organs, because anticoagulant activity remains intact of the heparin bonded with the high molecular compound which is also stable for a long period of time.

Thus, the present invention is utilizable in a field of medical industry.

## Claims

1. A process for preparing an antithrombotic medical material comprising heparin bonded via an epoxy compound with two or more epoxy groups with a high molecular compound containing a functional group capable of bonding with an epoxy group wherein an amino group in the heparin is bonded with the epoxy group in the epoxy compound and the functional group capable of bonding with the epoxy group in the high molecular compound is bonded with epoxy group in the epoxy compound, which comprises:
a) reacting the amino group in heparin with the epoxy group in the epoxy compound in an aqueous solution at a pH not lower than 7; and
b) reacting the functional group capable of bonding with an epoxy group in the high molecular compound with an epoxy group in the heparin derivative obtained in step a), said heparin derivative being beforehand immersed in a liquid medium, unreacted epoxy group(s) remaining in the reaction product.

2. The process for preparing the antithrombotic medical material according to claim 1, wherein the functional group capable of bonding with the epoxy group is at least any one of a hydroxyl group, an amino group, a carboxyl group and a mercapto group.

3. The process for preparing the antithrombotic medical material according to claim 1 or 2 wherein the liquid medium is a buffer solution.

4. The process for preparing the antithrombotic medical material according to claim 3 wherein the buffer solution is an aqueous phosphate or borate solution.

5. The process for preparing the antithrombotic medical material according to claim 1 wherein the liquid medium is an aqueous solution of an inert hydrophilic organic solvent.

6. A process for preparing a heparin derivative produced by reacting heparin with an epoxy compound containing two or more epoxy groups comprising a bond between the amino group of the heparin and the epoxy group of the epoxy compound, unreacted epoxy groups remaining in the reaction product, which comprises reacting heparin with an epoxy compound with two or more epoxy groups in a liquid medium at a pH not lower than 7.

7. The process according to claim 6, wherein the pH is between 8 and 10.

8. The process according to claim 6, wherein the reaction is conducted in an aqueous solution at pH 9 at room temperature.

9. The process according to claim 6, wherein the epoxy compound is 1,4-butanediol diglycidyl ether.

## Patentansprüche

1. Verfahren zur Herstellung eines antithrombotischen medizinischen Materials, umfassend über eine Epoxyverbindung mit zwei oder mehreren Epoxygruppen an eine hochmolekulare Verbindung mit einer funktionellen Gruppe mit der Fähigkeit zur Verbindung mit einer Epoxygruppe gebundenes Heparin, wobei eine Aminogruppe im Heparin mit der Epoxygruppe in der Epoxyverbindung und die funktionelle Gruppe mit der Fähigkeit zur Verbindung mit der Epoxygruppe in der hochmolekularen Verbindung mit einer Epoxygruppe in der Epoxyverbindung verbunden sind, durch
a) Umsetzen der Aminogruppe im Heparin mit der Epoxygruppe in der Epoxyverbindung in einer wäßrigen Lösung eines pH-Werts nicht unter 7 und
b) Umsetzen der in der hochmolekularen Verbindung vorhandenen funktionellen Gruppe mit der Fähigkeit zur Verbindung mit einer Epoxygruppe mit einer Epoxygruppe in dem in Stufe a) erhaltenen Heparinderivat, wobei das Heparinderivat zuvor in ein flüssiges Medium eingetaucht wird und im Reaktionsprodukt (eine) nicht umgesetzte Epoxygruppe(n) zurückbleibt(zurückbleiben).

2. Verfahren zur Herstellung des antithrombotischen medizinischen Materials nach Anspruch 1, wobei die funktionelle Gruppe mit der Fähigkeit zur Verbindung mit der Epoxygruppe aus mindestens einer Hydroxyl-, Amino-, Carboxyl- und/oder Mercaptogruppe besteht.

3. Verfahren zur Herstellung des antithrombotischen medizinischen Materials nach Anspruch 1 oder 2, wobei das flüssige Medium aus einer Pufferlösung besteht.

4. Verfahren zur Herstellung des antithrombotischen medizinischen Materials nach Anspruch 3, wobei die Pufferlösung aus einer wäßrigen Phosphat- oder Boratlösung besteht.

5. Verfahren zur Herstellung des antithrombotischen medizinischen Materials nach Anspruch 1, wobei das flüssige Medium aus einer wäßrigen Lösung eines inerten hydrophilen organischen Lösungsmittels besteht.

6. Verfahren zur Herstellung eines Heparinderivats, hergestellt durch Umsetzen von Heparin mit einer Epoxyverbindung mit zwei oder mehreren Epoxygruppen, umfassend eine Bindung zwischen der Aminogruppe des Heparins und der Epoxygruppe der Epoxyverbindung, wobei nicht umgesetzte Epoxygruppen im Reaktionsprodukt verbleiben, durch Umsetzen von Heparin mit einer Epoxyverbindung mit zwei oder mehreren Epoxygruppen in einem flüssigen Medium bei einem pH-Wert nicht unter 7.

7. Verfahren nach Anspruch 6, wobei der pH-Wert zwischen 8 und 10 liegt.

8. Verfahren nach Anspruch 6, wobei die Umsetzung in einer wäßrigen Lösung bei einem pH-Wert von 9 bei Raumtemperatur durchgeführt wird.

9. Verfahren nach Anspruch 6, wobei die Epoxyverbindung aus 1,4-Butandioldiglycidylether besteht.

## Revendications

1. Procédé de préparation d'une matière médicale antithrombotique comprenant de l'héparine liée par l'intermédiaire d'un composé époxy comportant deux groupements époxy ou plus à un composé de poids moléculaire élevé contenant un groupement fonctionnel capable de se lier à un groupement époxy, dans lequel un groupement amino de l'héparine est lié au groupement époxy du composé époxy et le groupement fonctionnel du composé de poids moléculaire élevé, capable de se lier au groupement époxy, est lié au groupement époxy du composé époxy, lequel procédé comprend :
a) la réaction du groupement amino de l'héparine avec le groupement époxy du composé époxy dans une solution aqueuse à un pH supérieur ou égal à 7; et
b) la réaction du groupement fonctionnel du composé de poids moléculaire élevé, capable de se lier à un groupement époxy, avec un groupement époxy du dérivé d'héparine obtenu à l'étape a), ledit dérivé d'héparine étant préalablement immergé dans un milieu liquide, le ou les groupements époxy n'ayant pas réagi demeurant dans le produit réactionnel.

2. Procédé de préparation de la matière médicale antithrombotique selon la revendication 1, dans lequel le groupement fonctionnel capable de se lier au groupement époxy est au moins l'un quelconque des groupements hydroxyle, amino, carboxyle et mercapto.

3. Procédé de préparation de la matière médicale antithrombotique selon la revendication 1 ou 2, dans lequel le milieu liquide est une solution tampon.

4. Procédé de préparation de la matière médicale antithrombotique selon la revendication 3, dans lequel la solution tampon est une solution aqueuse de phosphate ou borate.

5. Procédé de préparation de la matière médicale antithrombotique selon la revendication 1, dans lequel le milieu liquide est une solution aqueuse d'un solvant organique hydrophile inerte.

6. Procédé de préparation d'un dérivé d'héparine produit par réaction de l'héparine avec un composé époxy contenant deux groupements époxy ou plus comprenant une liaison entre le groupement amino de l'héparine et le groupement époxy du composé époxy, les groupements époxy n'ayant pas réagi demeurant dans le produit réactionnel, lequel procédé comprend la réaction de l'héparine avec un composé époxy comportant deux groupements époxy ou plus dans un milieu liquide à un pH supérieur ou égal à 7.

7. Procédé selon la revendication 6, dans lequel le pH est compris entre 8 et 10.

8. Procédé selon la revendication 6, dans lequel la réaction est conduite dans une solution aqueuse à un pH de 9 à la température ambiante.

9. Procédé selon la revendication 6, dans lequel le composé époxy est un éther diglycidylique de 1,4-butanediol.
